(19) Europäisches Patentamt
European Patent Office
Office européen des brevets

(11) Veröffentlichungsnummer: **0 268 073**
**A1**

# (12) EUROPÄISCHE PATENTANMELDUNG

(21) Anmeldenummer: **87114986.0**

(22) Anmeldetag: **14.10.87**

(51) Int. Cl.⁴: **C07C 69/017** , C07C 69/16 , C07C 69/78 , C07C 43/295 , C07C 39/15 , C07C 67/42

(30) Priorität: **28.10.86 DE 3636561**

(43) Veröffentlichungstag der Anmeldung: **25.05.88 Patentblatt 88/21**

(84) Benannte Vertragsstaaten: **BE DE ES FR GB**

(71) Anmelder: **Röhm GmbH**
**Kirschenallee Postfach 4242**
**D-6100 Darmstadt 1(DE)**

(72) Erfinder: **Knebel, Joachim, Dr.**
**Dieselstrasse 20**
**D-6100 Darmstadt(DE)**
Erfinder: **Ude, Werner, Dr.**
**Birngartenweg 115**
**D-6100 Darmstadt-Arheilgen(DE)**

## (54) Verfahren zur Herstellung von Dihydroxi- und Diacyloxidiphenylen.

(57) Die Erfindung betrifft die Einführung von phenolischem Sauerstoff in die 4,4′-Positionen von Diphenylether und/oder von Biphenyl durch Oxidation der 4,4′-Diacylverbindungen mit Persäuren zu den entsprechenden 4,4′-Diacyloxiverbindungen, die isoliert und weiter zu 4,4′-Dihydroxidiphenylether oder/und 4,4′-Dihydroxibiphenyl hydrolysiert werden können.

EP 0 268 073 A1

## Gebiet der Erfindung

Die Erfindung betrifft ein Verfahren zur Herstellung von 4,4'-Dihydroxidiphenylether oder von 4,4'-Dihydroxibiphenyl bzw. Gemische derselben und ein Verfahren zur Herstellung der entsprechenden 4,4'-Diacyloxiverbindungen als deren Vorstufen aus Diphenylether oder aus Biphenyl bzw. aus deren Gemischen.

### Stand der Technik

Aromatische Dihydroxiverbindungen sind als bifunktionelle Verbindungen für die Herstellung von Kunststoffen interessant. Die weitaus wichtigste Verbindung aus der Reihe der Dihydroxidiphenyle für die technische Herstellung von Kunststoffharzen ist 2,2-Bis-(4-hydroxyphenyl)-propan.
Einer technischen Verwendung von 4,4'-Dihydroxidiphenylether fur die Herstellung von Polykondensationsharzen mit vorteilhaften Eigenschaften, stehen unbefriedigende Herstellungsverfahren dieses Grundbausteins entgegen.

Nach Metody Poluch.Khim. Reaktiv. Prep. 1971, Nr. 23, 54 - 55 (Ref.: CA. 78, 2938 u) wird 4,4'-Dihydroxidiphenylether in einer Ausbeute von 51,5 % durch Diazotierung von über mehrere Stufen aufwendig hergestelltem 4,4'-Diaminodiphenylether und anschließender Zersetzung des Diazoniumsalzes mit Schwefelsäure erhalten.

In der US-PS 3 290 386 ist ein Verfahren zur Herstellung von 4,4'-Dihydroxidiphenylether ausgehend von 4,4'-Dibromdiphenylether beschrieben, wobei dieser mit Natronlauge in Gegenwart von Kupfer (I)-Ionen und Natriumperoxid bei 185 bis 190 Grad C hydrolysiert wird. Nach der Neutralisation mit Salzsäure wird das Verfahrensprodukt in nahezu quantitativer Ausbeute erhalten.

Die Hydrolyse liefert bei diesem Verfahren neben dem gewünschten Produkt als Beiprodukt Natriumbromid in wäßriger Lösung. Dieses und die bei der Herstellung der Ausgangsverbindungen durch Bromierung von Diphenylether entstehende Bromwasserstoffsäure müssen wieder als Brom in das Verfahren zurückgeführt werden.

In der deutschen Anmeldung P 35 06 845.0 wird ein Verfahren zur Herstellung von 4,4'-Diyhdroxydiphenylether über die entsprechende 4,4'-Dijodverbindung beschrieben, wodurch die schwierigeren Handhabungen mit korrosiver Bromwasserstoffsäure und korrosiven Broms vermieden werden.

Eine Herstellung von 4,4'-Dihydroxidiphenylether durch Alkalischmelzen entsprechender 4,4'-Disulfonsäureverbindungen, wie dies bei der Herstellung von 4,4'-Dihydroxibiphenyl entsprechend technisch durchgeführt wird, läßt sich wegen der dabei auftretenden Diphenyletherspaltung nicht bewerkstelligen.

Der große Stand der Technik zur Herstellung von 4,4'-Dihydroxibiphenyl durch Alkalischmelze von 4,4'-Disulfonsäuren-bzw. deren Salzen des Biphenyls, wie er in der DE-A 32 04 079, in der DE-A 32 26 391 oder in der US-PS 4 243 822 angegeben ist, zeigt auch, daß diese Verfahren trotz billiger Ausgangssubstanzen nicht befriedigen.

### Aufgabe und Lösung

Es bestand daher die Aufgabe, für die Herstellung von 4,4'-Dihydroxidiphenylether aus dem preisgünstigen Ausgangsprodukt Diphenylether eine technisch einfache und wirtschaftliche Synthese zu finden.

Es wurde gefunden, daß 4,4-Dihydroxidiphenylether in hohen Ausbeuten erhalten wird, wenn eine 4,4'-Diacylverbindung des Diphenylethers mit einer Persäure in die 4,4'-Diacyloxiverbindung überführt und diese anschließend hydrolysiert wird. Aus dem gegebenenfalls dabei anfallenden Salz wird durch Ansäuern die freie Dihydroxiverbindung gebildet.

Als technisch besonders preisgünstige Ausgangsverbindung haben sich Gemische von Diphenylether mit Biphenyl, insbesondere das azeotrope Gemisch aus 73 Gew.-% Diphenylether und 27 Gew.-% Biphenyl, wie es beispielsweise als Diphyl ® im Handel ist, erwiesen. Dabei wurde überraschenderweise weiter gefunden, daß neben dem Diphenylether, das Biphenyl über die Stufe der entsprechenden Diacylverbindung in hohen Ausbeuten zum 4,4'-Diacyloxibiphenyl und gegebenenfalls daraus weiter zu dem 4,4'-Dihydroxibiphenyl umgesetzt wird. 4,4'-Dihydroxibiphenyl wird wie weiter gefunden wurde, auch durch Umsetzung von reinem Biphenyl uber die angegebenen Zwischenstufen in hoher Ausbeute erhalten.

Die Erfindung betrifft ein Verfahren zur Herstellung einer 4,4'-Dihydroxidiphenylverbindung der Formel

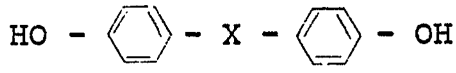,

in der X = O oder die Biphenylverknüpfung darstellt, aus einer eintsprechenden Diphenylverbindung der Formel

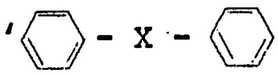

das dadurch gekennzeichnet ist, daß

a) die Diphenylverbindung zur Diacyldiphenylverbindung acyliert wird,

b) die Diacyldiphenylverbindung mit einer Persäure zur Diacylixidiphenylverbindung oxidiert wird,

c) die Diacyloxidiphenylverbindung hydrolysiert wird und gegebenenfalls

d) die Dihydroxidiphenylverbindung durch Säurezugabe freigesetzt wird.

In den erfindungsgemäßen Verfahren zur Herstellung von 4,4′-Dihydroxidiphenylether oder/und 4,4′-Dihydroxibiphenyl ist der oxidative Schritt, wie er oben unter b) formuliert ist, für die Herstellung der entsprechenden Diacyloxiverbindungen neu. Dieser Schritt ist zur Herstellung der Dihydroxiverbindungen von Diphenylether und Biphenyl bei dem erfindungsgemäßen Verfahren wesentlich, da hierbei die gewünschte Sauerstoffunktion gebildet wird.

Vorteile der Erfindung

Durch die erfindungsgemäße Herstellung der Diacyloxiverbindungen von Diphenylether und/oder Biphenyl durch Oxidation der entsprechenden Diacylverbindungen, ist gegenuber den bisherigen Möglichkeiten zur Herstellung des insbesondere wichtigen und wertvollen 4,4′-Dihydroxidiphenylethers ein technisch wesentlich besserer und wirtschaftlicherer Weg gegeben. Gegenüber den Verfahren des Standes der Technik, die uber Brom-und Iodverbindungen laufen, entfällt hier das Arbeiten mit diesen aggressiven und/oder teuren Halogenen bzw. Halogenverbindungen.

Die bei dem erfindungsgemäßen Verfahren gebildeten Zwischenprodukte, wie die bei der Oxidation der Diacylverbindungen entstehenden Diacyloxiverbindungen wie z. B. 4,4′-Diacetoxidiphenylether und/oder 4,4′-Diacetoxibiphenyl, oder die gegebenenfalls durch alkalische Hydrolyse der Diacyloxiverbindungen entstehenden phenolischen Salze, z.B. das Dinatriumsalz von 4,4′-Dihydroxidiphenylether, können auch als solche isoliert werden. Diese Verbindungen stellen wertvolle Produkte dar und sind insbesondere als reaktive Zwischenprodukte, z.B. bei Polykondensationsreaktionen wichtig.

Auch die Herstellung von bifunktionellen Gemischen aus Diacyloxi-oder Dihydroxiverbindungen, bzw. deren Salzen, durch Verwendung von Diphenylether/Biphenyl-Ausgangs-Gemischen, bringt neben einem Kostenvorteil auch die Möglichkeit, insbesondere bei Polykondensationsreaktionen, Eigenschaften von Polymeren durch Verwendung von reaktiven Gemischen entsprechend einzustellen.

Durchführung der Erfindung

Diphenylether und Biphenyl und Gemische derselben sind technische Produkte.

Die Acylierung

dieser aromatischen Verbindungen mit aliphatischen oder aromatischen Carbonsäurederivaten zu den Diacylverbindungen, vornehmlich zu den 4,4′-Diacylverbindungen, wird durch Friedel-Crafts-Acylierung wie beispielsweise in Ullmanns Encyclopädie der technischen Chemie, 4. Auflage, Band 7, Seiten 101 bis 103 beschrieben, durchgeführt. Insbesondere werden aliphatische Acylierungsmittel, bevorzugt Acetylderivate, wie das Acetylchlorid, eingesetzt.

Beispielsweise ist im Journal of Org. Chem. (USSR) 3,1005 (1967) die Herstellung von 4,4′-Diacetodiphenylether in 92 bis 96 %-iger Ausbeute aus Diphenylether und Acetylchlorid mit $AlCl_3$ als Katalysator beschrieben. Acylierungen auch mit weiteren Säurechloriden, nämlich Propionsäure-oder Buttersäurechlorid, sowohl von Diphenyl als auch von Diphenylether, sind in Makromol. Chem. 49, 62 bis 65 (1961) angegeben.

Die Oxidation

der nach der Acylierung vorliegenden Ketone zu den entsprechenden Acyloxiphenylverbindungen, d.h. zu den Phenolestern der als Ausgangsprodukte eingesetzten Phenylverbindungen, wird mit Hilfe von Persäuren nach Art der Baeyer-Villiger-Reaktion durchgeführt. Das erfindungsgemäße Verfahren zur Herstellung von 4,4'-Diacyloxiverbindungen der Formel I

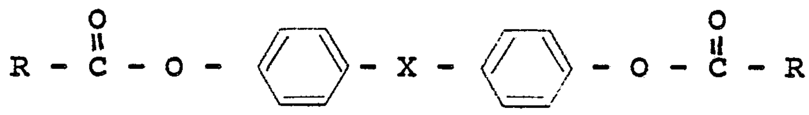

I

durch Oxidation von 4,4'-Diacylverbindungen der Formel II

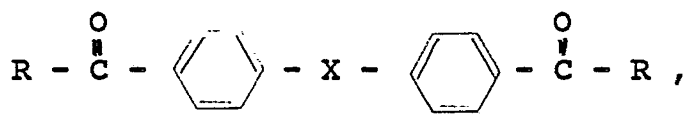

II

in denen

R Alkyl mit 1 bis 15 C-Atomen oder Aryl, insbesondere Phenyl und

X eine Einfachbindung oder ein Sauerstoffatom

bedeuten, ist neu.

Das neue Verfahren für die Herstellung der Verbindungen I aus den Verbindungen wird unter Bedingungen wie sie fur die Bayer-Villiger-Reaktion bekannt und beispielsweise in Org. React. 9 (1957), 73 - 107 beschrieben sind, ausgeführt. Die Oxidation wird mit Persäuren in inerten organischen Lösungsmitteln, wie Methylenchlorid, 1,2-Dichloräthan, Acetonitril, Toluol, gegebenenfalls in Gegenwart von starken Säuren, wie Schwefelsäure, p-Toluolsulfonsäure, Methansulfonsäure, Trifluoressigsäure, bei Temperaturen im Bereich von -20 Grad C bis etwa 50 Grad C durchgeführt. Als Oxidationsmittel sind anorganische und/oder organische Persäuren wie beispielsweise Perschwefelsäure, $H_2SO_5$ oder Permonophosphorsäure $H_3PO_5$ oder Trifluorperessigsäure bekannt und brauchbar. Besonders vorteilhaft hat sich die Verwendung von Permonophosphorsäure, $H_3PO_5$, als Oxidationsmittel erwiesen. Damit werden in kurzen Reaktionszeiten, beispielsweise in 1 Stunde, Ausbeuten von 96 % d. Th. an 4,4'-Diacetoxidiphenylether erhalten.

Nach bekannten Aufarbeitungsmethoden können auf dieser Stufe die Reaktionsprodukte, die 4,4'-Diacyloxidiphenylether bzw. die 4,4'-Diacyloxibiphenyle, wie z.B. 4,4'-Diacetoxidiphenylether oder 4,4'-Diacetoxibiphenyl oder auch deren Gemische isoliert und als solche weiterverwendet werden. Es ist auch möglich, Gemische der Diacyloxiverbindungen von Diphenylether und Biphenyl in die Diacyloxiverbindungen des Diphenylethers und des Biphenyls, z.B. durch fraktionierte Kristallisationen, zu trennen.

Die Hydrolyse

der 4,4'-Diacyloxiverbindungen kann im alkalischen wie im sauren Medium, nach Methoden wie sie beispielsweise in Houben-Weyl, Methoden der organischen Chemie, 4. Auflage. Bd. VI/1c, 436 ff, beschrieben sind, durchgeführt werden. Bei der sauren Hydrolyse werden dabei 4,4'-Dihydroxidiphenylether oder 4,4'-Dihydroxibiphenyl oder deren Gemische gebildet, die gegebenenfalls auch aus den bei der alkalischen Hydrolyse zunächst gebildeten phenolischen Salzen durch Ansäuern ebenfalls erhalten werden können.

Die Verfahrensprodukte wie beispielsweise 4,4'-Diacetoxidiphenylether mit Fp 109 bis 111 Grad C, 4,4'-Diacetoxibiphenyl mit Fp 162,5 Grad C, und 4,4'-Dihydroxidiphenylether mit Fp 165 Grad C oder 4,4'-Dihydroxibiphenyl lassen sich insbesondere durch Kristallisation reinigen und trennen.

BEISPIELE

Beispiel 1

12,7 g (0,05 mol) 4,4'-Diacetyldiphenylether werden in 20 ml Acetonitril aufgeschlämmt und auf 0 Grad C gekühlt. Bei dieser Temperatur tropft man 0,2 mol Monoperphosphorsäure in Acetonitril (dargestellt nach Y. Ogata et al., J. Org. Chem. 43 (12), 2417 (1978)) zu und erwärmt auf Raumtemperatur. Man rührt eine Stunde nach, gießt dann die Reaktionsmischung in überschüssiges Wasser und zerstört restliche Perverbindungen durch Zugabe von $NaHSO_3$. Der ausgefallene 4,4'-Diacetoxidiphenylether wird abfiltriert und in einer Mischung aus 70 ml Wasser und 20 ml Ethanol suspendiert. Man gibt 28 g (0,4 mol) Kaliumhydroxid hinzu und erhitzt 2 Stunden am Rückfluß. Dann läßt man abkühlen, extrahiert die Reaktionsmischung zweimal mit je 50 ml $CH_2Cl_2$, trennt die wässrige Phase ab und säuert diese an. Das ausgefallene Produkt wird abgesaugt, mit kaltem Wasser gewaschen und im Vakuum getrocknet. Es wird massenspektroskopisch als 4,4'-Dihydroxidiphenylether identifiziert.

Ausbeute

9,7 g 4,4'-Dihydroxidiphenylether (96 % d. Th. bezogen auf 4,4'-Diacetyldiphenylether).

Beispiel 2

Wie Beispiel 1, jedoch unter Verwendung von 11,9 g (0,05 mol) 4,4'-Diacetylbiphenyl.
Reaktionszeit der Oxidation: 4 Stunden bei Raumtemperatur.

Ausbeute

9 g 4,4'-Dihydroxibiphenyl (97 % d.Th, bzogen auf 4,4'-Diacetylbiphenyl); identifiziert durch Massenspektroskopie.

Beispiel 3

6,35 g (0,025 mol) 4,4'-Diacetyldiphenylether werden in 200 ml Methylenchlorid gelöst und mit 47 g (0,33 mol) $Na_2HPO_4$ versetzt. Unter heftigem Rühren tropft man in 30 Minuten 0,075 mol $CF_3CO_3H$ in 25 ml $CH_2Cl_2$ (dargestellt nach M.F. Hawthorne et al., J. Am. Chem Soc. 80, 6396 (1958)) bei Raumtemperatur zu. Man rührt 15 Minuten nach, versetzt mit 200 ml Wasser und zerstört restliche Perverbindungen durch Zugabe von $NaHSO_3$. Man trennt die org. Phase ab, extrahiert die Wasserphase zweimal mit je 100 ml $CH_2Cl_2$ und engt die vereinigten organischen Anteile im Vakuum ein. Der verbleibende Ruckstand wird wie in Beispiel 1 mit ethanolischer KOH verseift.

Ausbeute

3,55 g 4,4'-Dihydroxidiphenylether, massenspektroskopisch identifiziert (70 % d. Th. bezogen auf 4,4'-Diacetyldiphenylether).

Beispiel 4

12,7 g (0,05 mol) Diacetyldiphenylether werden in 20 ml Eisessig aufgeschlämmt und auf 0 Grad C gekühlt. Bei dieser Temperatur tropft man in 45 Minuten 0,19 mol (37 g) 40 %-ige Peressigsäure zu, die 0,5 % p-Toluolsulfonsäure enthält. Man rührt 23 Stunden bei Raumtemperatur und arbeitet den Ansatz analog Beispiel 1 auf.

Ausbeute

5,5 g 4,4'-Dihydroxidiphenylether, durch Massenspektroskopie identifiziert (55 % d. Th. bezogen auf 4,4'-Diacetyldiphenylether).

Beispiel 5

91 g (0,68 mol) $AlCl_3$ werden in 140 ml Methylenchlorid suspendiert und bei 3 bis 7 Grad C mit 70 g (0,89 mol) Acetylchlorid versetzt. Die entstehende Aufschlämmung tropft man bei 0 bis 3 Grad C zu einer Lösung von 24,4 g (0,143 mol) Diphenylether und 8,8 g (0,057 mol) Biphenyl (entspricht der Zusammensetzung von Diphyl®) in 60 ml $CH_2Cl_2$, rührt das Reaktionsgemisch 1 Stunde bei -2 Grad C und anschließend 3 Stunden bei 40 Grad C (Rückfluß). Anschließend trägt man den Ansatz in ein Gemisch aus 500 g Eis und 25 ml konzentrierter Salzsäure ein und extrahiert mit Methylenchlorid. Das nach Einengen des Lösungsmittels erhaltene Rohprodukt wird aus Ethanol umkristallisiert.

Ausbeute

23,6 g eines Gemisches aus 4,4'-Diacetyldiphenylether und 4,4'-Diacetylbiphenyl,
Schmelzbereich 85 bis 149 Grad C.

12,5 g (0,05 mol) des obigen Acetylierungsprodukts werden in 30 ml Acetonitril gelöst und innerhalb von zwei Stunden bei 10 Grad C mit 0,2 mol Monoperphosphorsäure in Acetonitril versetzt. Man rührt 5 Stunden bei Raumtemperatur nach, gießt dann die Reaktionsmischung in überschüssiges Wasser und zerstört restliche Perverbindungen durch Zugabe von $NaHSO_3$. Durch Extraktion mit Methylenchlorid und nachfolgendem Einengen erhält man das Reaktionsprodukt, bestehend aus 4,4'-Diacetoxidiphenylether und 4,4'-Diacetoxibiphenyl. Es wird in einer Mischung aus 70 ml Wasser und 20 ml Ethanol suspendiert. Man gibt 27 g (0,39 mol) Kaliumhydroxid hinzu und erhitzt 1,5 Stunden am Rückfluß. Dann läßt man abkühlen, extrahiert die Reaktionsmischung zweimal mit je 50 ml $CH_2Cl_2$, trennt die wäßrige Phase ab und säuert diese an. Das ausgefallene Produkt wird abgesaugt und im Vakuum getrocknet.

Ausbeute

5 g eines Gemisches, das nach massenspektroskopischen Untersuchungen und nach GC-Analysen aus 57 Gew.-% 4,4'-Dihydroxidiphenylether und 43 Gew.-% 4,4'-Dihydroxibiphenyl besteht.
Schmelzbereich: 187 bis 189 Grad C.

## Ansprüche

1. Verfahren zur Herstellung einer 4,4'-Dihydroxidiphenylverbindung der Formel

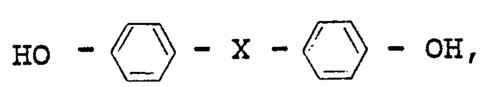

in der X = O oder die Biphenylverknüpfung darstellt, aus einer entsprechenden Diphenylverbindung der Formel

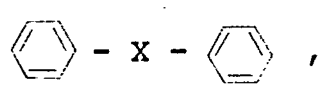

dadurch gekennzeichnet,
daß

a) die Diphenylverbindung zur Diacyldiphenylverbindung acyliert wird,

b) die Diacyldiphenylverbindung mit einer Persäure zur Diacyloxidiphenylverbindung oxidiert wird,

c) die Diacyloxidiphenylverbindung hydrolysiert wird und gegebenenfalls

d) die Dihydroxidiphenylverbindung durch Säurezugabe freigesetzt wird.

2. Verfahren nach Anspruch 1, dadurch gekennzeichnet, daß die Acylierung nach a) nach Friedel-Crafts, vorzugsweise mit $AlCl_3$ durchgeführt wird.

3. Verfahren nach den Ansprüchen 1 und 2, dadurch gekennzeichnet, daß die Acylierung mit aliphatischen Acylierungsmitteln durchgeführt wird.

4. Verfahren nach den Ansprüchen 1 bis 3, dadurch gekennzeichnet, daß die Acylierung mit Acetylverbindungen, insbesondere mit Acetylchlorid, durchgeführt wird.

5. Verfahren nach den Ansprüchen 1 und 2, dadurch gekennzeichnet, daß die Acylierung mit aromatischen Acylverbindungen durchgeführt wird.

6. Verfahren nach den Ansprüchen 1 bis 5, dadurch gekennzeichnet, daß die Oxidation der Diacyldiphenylverbindungen mit einer anorganischen Persäure durchgeführt wird.

7. Verfahren nach den Ansprüchen 1 bis 6, dadurch gekennzeichnet, daß die Oxidation mit Permonophosphorsäure, $H_3PO_5$, durchgeführt wird.

8. Verfahren zur Herstellung von 4,4'-Diacyloxidiphenylethern, dadurch gekennzeichnet, daß sie durch Oxidation der entsprechenden 4,4'-Diacylverbindungen mit einer Persäure hergestellt werden.

9. Verfahren nach Anspruch 8, dadurch gekennzeichnet, daß als Acylverbindung die Acetylverbindung oxidiert wird.

10. Verfahren nach Anspruch 8, dadurch gekennzeichnet, daß als Acylverbindung die Benzoylverbindung oxidiert wird.

11. Verfahren nach den Ansprüchen 8 bis 10, dadurch gekennzeichnet, daß die Oxidation mit einer anorganischen Persäure durchgeführt wird.

12. Verfahren nach den Ansprüchen 8 bis 11, dadurch gekennzeichnet, daß die Oxidation mit Permonophosphorsäure, $H_3PO_5$, durchgeführt wird.

13. Verfahren nach den Ansprüchen 1 bis 12, dadurch gekennzeichnet, daß als Ausgangsmaterial zur Herstellung der 4,4'-Dihydroxidiphenylverbindungen ein Gemisch von Diphenylether und Biphenyl verwendet wird

Europäisches Patentamt

# EUROPÄISCHER RECHERCHENBERICHT

Nummer der Anmeldung

EP 87 11 4986

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | THE JOURNAL OF ORGANIC CHEMISTRY, Band 43, Nr. 12, 9. Juni 1978, Seiten 2417-2419, American Chemical Society, US; Y. OGATA: "Kinetics of Baeyer-Villiger reaction of acetophenones with permonophosphoric acid"<br>* Insgesamt *<br>--- | 1-13 | C 07 C 69/017<br>C 07 C 69/16<br>C 07 C 69/78<br>C 07 C 43/295<br>C 07 C 39/15<br>C 07 C 67/42 |
| Y | JOURNAL OF THE CHEMICAL SOCIETY, Sektion C, 1967, Seiten 272-274, American Chemical Society, US; J.M. BLATCHLY et al.: "Biphenylenes. Part XVI. Preparation and Baeyer-Villiger oxidation of some 2-acetylbiphenylenes"<br>* Insgesamt *<br>----- | 1-13 | |
| | | | **RECHERCHIERTE SACHGEBIETE (Int. Cl.4)** |
| | | | C 07 C 67/00<br>C 07 C 69/00 |

Der vorliegende Recherchenbericht wurde für alle Patentansprüche erstellt

| Recherchenort | Abschlußdatum der Recherche | Prüfer |
|---|---|---|
| DEN HAAG | 04-02-1988 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument